## Europäisches Patentamt

## European Patent Office

## Office européen des brevets

(11) Veröffentlichungsnummer: **0 136 393**
**B1**

(12) **EUROPÄISCHE PATENTSCHRIFT**

(45) Veröffentlichungstag der Patentschrift:
**22.06.88**

(21) Anmeldenummer: **84101264.4**

(22) Anmeldetag: **08.02.84**

(51) Int. Cl.⁴: **A 61 N 1/42, A 61 F 13/02**

(54) Magnetfolien.

(30) Priorität: **05.10.83 DE 3336166**
**04.11.83 DE 8331666 U**

(43) Veröffentlichungstag der Anmeldung:
**10.04.85 Patentblatt 85/15**

(45) Bekanntmachung des Hinweises auf die Patenterteilung:
**22.06.88 Patentblatt 88/25**

(84) Benannte Vertragsstaaten:
**AT BE CH DE FR GB IT LI LU NL SE**

(56) Entgegenhaltungen:
**EP - A - 0 076 354**
**EP - A - 0 081 109**
**DE - A - 1 900 744**
**DE - A - 2 301 010**
**DE - A - 2 914 904**
**DE - B - 1 161 384**
**FR - A - 1 457 985**
**GB - A - 1 599 721**
**GB - A - 2 052 993**

(73) Patentinhaber: **LACOTHERM AG, Kronenstrasse 191,
CH-9427 Wolfhalden (CH)**

(72) Erfinder: **Latzke, Arno Walter, Mühltobel 946,
CH-9429 Zelg/Wolfhalden (CH)**

(74) Vertreter: **Werner, Hans-Karsten, Dr. et al,
Deichmannhaus am Hauptbahnhof, D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind Magnetfolien mit streifenförmiger abwechselnd positiver und negativer Magnetisierung in Abständen von 1 bis 12 mm, vorzugsweise 3 bis 6 mm mit Feldstärken von 300 bis 1000 Gauss und ein Verfahren zur Befestigung derselben, insbesondere an bewegten und/oder behaarten Körperteilen.

Es ist bekannt, die therapeutische Wirkung von Magnetfeldern einzusetzen mit Hilfe aufwendiger Apparaturen oder durch magnetische Heilpflaster, die aus einem Ferritplättchen und einem selbstklebenden Heftpflaster bestehen. Derartige magnetische Heilpflaster sind beispielsweise aus dem deutschen Gebrauchsmuster 7 919 808 bekannt. Der Nachteil dieser magnetischen Heilpflaster besteht darin, dass sie ihre Wirkung nur dann entfalten wenn sie an den richtigen Stellen aufgeklebt werden. Das Auffinden der optimalen Befestigungspunkte ist insbesondere für den Laien, aber auch für den weniger routinierten Heilpraktiker oder Arzt mit Schwierigkeiten verbunden.

Aus dem deutschen Gebrauchsmuster 8 128 911 (entsprechend EP-A2-0 076 354) sind Magnetpflaster bekannt, welche aus einer 0,2 bis 2 mm starken, flexiblen, hautverträglichen, permanent magnetisierbaren und magnetisierten Kunststoff-Folie bestehen, welche entweder selbstklebend ist oder mit Hilfe von hautverträglichen, selbstklebendem Pflaster auf der Haut befestigt wird. Der Nachteil derartiger Magnetpflaster ist, dass die Behandlungsfläche noch immer relativ klein ist, da sich grossflächige Pflaster nur unbequem tragen lassen und insbesondere an behaarten Körperteilen entweder schlecht haften oder sich nur sehr unangenehm wieder entfernen lassen.

Aus dem deutschen Gebrauchsmuster 8 313 968 sind hautverträgliche Pflaster zur Befestigung von Magnetfolien bekannt, welche geeignet sind, Magnetfolien mehrfach zur Anwendung zu bringen. Aber auch diese Befestigung hat noch immer den Nachteil, dass sie an bewegten und/oder behaarten Körperteilen schlecht haftet oder schlecht wieder zu entfernen ist. Ein weiterer Nachteil aller bisher bekannten selbstklebenden oder aufgeklebten Magnetfolien ist, dass sie bei längerer und mehrfacher Verwendung brechen und dadurch unbrauchbar werden.

Es ist weiterhin bekannt, dass feuchte Wärme eine heilende und lindernde Wirkung hat. Dies wird bei der Therapie mit feuchten Umschlägen sowie elektrisch beheizten Flanellkissen ausgenutzt. Der Nachteil dieser Behandlungsmethoden besteht darin, dass sie in der Anwendung unpraktisch sind, da man entweder die Umschläge mit wasserabdichtenden weiteren Schichten versehen muss oder aber bei dem Heizkissen teure und nicht ganz ungefährliche elektrische Einrichtungen benötigt.

Die Erfindung hat sich die Aufgabe gestellt, an sich bekannte Magnetfolien an bewegten und oder behaarten Körperteilen zu befestigen, so dass sie gut haften und sich gut entfernen lassen, mehrfach anwendbar sind und eine längere Lebensdauer aufweisen.

Die Erfindung hat sich die weitere Aufgabe gestellt, die Behandlung mit feuchter Wärme zu verbessern und zu vereinfachen.

Die oben genannten Aufgaben lassen sich überraschenderweise dadurch lösen, dass man Magnetfolien mit streifenförmiger abwechselnder positiver und negativer Magnetisierung in Abständen von 1 bis 12 mm, vorzugsweise 3 bis 6 mm mit Feldstärken von 300 bis 1000 Gauss verwendet, die dadurch gekennzeichnet sind, dass sie mindestens auf einer Seite ein Textilgewebe aus saugfähigem, hautverträglichem Textilgewebe aus Wolle, Baumwolle oder Leinen aufweisen. Dieses Textilgewebe wird anstelle der bisherigen selbstklebenden Schichten auf die Haut gebracht und vermeidet dadurch den direkten Kontakt der Magnetfolie oder der Klebschichten mit der Haut. Das Textilgewebe speichert darüberhinaus Feuchtigkeit, die zusammen mit der Erwärmung durch die Magnetfolie die Behandlung mit feuchter Wärme ermöglicht.

Gewünschtenfalls können in dieses Gewebe, insbesondere wenn es stark saugfähig ist, ausser Wasser auch Salben, Crèmes, Linimente und Fluids eingebracht werden, die zusammen mit der Magnetfolie und der feuchten Wärme eine verstärkte Wirkung entwickeln.

Da das direkte Aufkleben der Magnetfolie auf der Haut entfällt, treten keine Spannungen mehr zwischen Haut und Folie auf, so dass die gelegentlich beobachteten Allergien und Entzündungen durch Reibung, insbesondere an den Folienkanten und Bruchstellen der Folie von vornherein vermieden werden.

Insbesondere wenn das erfindungsgemäss verwendete Gewebe abnehmbar ist, kann es in besonders einfacher Weise ausgetauscht oder gewaschen und hygienisch wieder verwendet werden.

Durch die häufige Wiederverwendbarkeit insbesondere des magnetischen Materials sinken auch die Kosten für die Anwendung gegenüber selbstklebenden Folien um ein Mehrfaches. Eine spezielle Ausführungsform der erfindungsgemässen Magnetfolien sind grossflächige Folien mit einer Fläche von 400 bis 1500 cm$^2$, die allseitig mit einem oder mehreren Textilgeweben überzogen sind, von den mindestens eines abnehmbar und waschbar ist. Diese grossflächigen Magnetfolien können einfach und in hygienischer Weise und trotzdem hoher Wirksamkeit mit den zu behandelnden Körperteilen in Berührung gebracht werden, indem man sie wie ein Kissen mit dem Körper in Berührung bringt. Während die bisher bekannten Magnetpflaster für mehrere Tage auf die zu behandelnden Stellen aufgeklebt werden mussten, genügt es bei den erfindungsgemässen grossflächigen Magnetfolien, dass sie stundenweise mit den zu behandelnden Körperteilen in Berührung gebracht werden. Sie können daher ähnlich einem Kissen im Bett auf Stühlen und Sesseln und Autositzen etc. zur Anwendung kommen. Die erfindungsgemässen grossflächigen Magnetfolien können im Prinzip beliebige Formen haben, vorzugsweise sind sie jedoch rund, oval, quadratisch oder rechteckig. Die erfindungsgemässen grossflächigen Magnetfolien sind von der Grösse her nicht beschränkt, jedoch sollte ihre Fläche aus praktischen Gründen im Bereich von 400 bis 1500 cm² liegen. Bei Flächen unter 400 cm² ist bei Kissen nicht mehr ohne weiteres gewährleistet, dass die zu behandelnden Körperteile mit den Magnetfeldern der Magnetfolie ausgesetzt sind. Flächen über 1500 cm² sind unhandlich und deshalb ebenfalls weniger bevorzugt. Eine typische bevorzugte Ausführungsform ist daher rechteckig und hat Kantenlängen von 25×40 cm. Die erfindungsgemässen grossflächigen Magnetfolien sollten Feldstärken von 300 bis 1000, vorzugsweise 400 bis 800 Gauss aufweisen, da nur bei derartigen Feldstärken durch das Textilgewebe hindurch noch ausreichende magnetische Feldstärkung zur Wirkung kommen können.

Erfindungsgemäss sollen die grossflächigen Magnetfolien mit mindestens einem abnehmbaren und waschbaren Textilgewebe überzogen sein, welches beispielsweise durch Knöpfe, einen Reissverschluss oder einen Klettverschluss verschliessbar ist. Selbstverständlich sind auch andere Verschlussarten wie Einschlaglaschen, Schnürenverschlüsse, Haken und Ösen etc. denkbar, die einerseits leicht handhabbar sind und andererseits bei der Benutzung von Magnetfolien wenig stören. Weiterhin kann der Kissenbezug auch mit einem Einschlag versehen werden, so dass auf einen weiteren Verschluss verzichtet werden kann.

Das abnehmbare und waschbare Textilgewebe sollte aus Wolle, Baumwolle oder Leinen bestehen, da diese besonders hautverträglich sind. Da die erfindungsgemässen grossflächigen Magnetfolien überwiegend nach Art eines Kissens benutzt werden, wird es als besonders angenehm empfunden, wenn ausser dem abnehmbaren waschbaren Textilgewebe eine weitere dünne Textilschicht vorhanden ist. Beispielsweise hat sich das Aufkleben, Einnähen oder Einschweissen der Magnetfolien in Flanellgewebe besonders bewährt.

Die Dicke der erfindungsgemässen grossflächigen Magnetfolien ist nicht kritisch, sofern die Dicke ausreicht, die notwendigen Feldstärken zu erzeugen. Weiterhin ist darauf zu achten, dass die verwendeten Magnetfolien mechanisch stabil genug sind, um bei der Verwendung in Form eines Kissens nicht zu leicht zu knicken und zu brechen. Schichtdicken von 1,5 bis 4 mm sind daher besonders geeignet.

Da handelsübliche Magnetfolien im allgemeinen nur einseitig magnetisiert sind und daher nur nach einer Seite hin starke Magnetfelder aufweisen, ist es für die praktische Handhabung sehr nützlich, wenn sowohl die Folie als auch das abnehmbare und waschbare Textilgewebe optisch markiert sind. Sofern die Magnetfolien noch mit einem zweiten Textilgewebe dauernd umgeben ist, reicht es selbstverständlich aus, dieses entsprechend zu kennzeichnen. Für die grossflächigen Magnetfolien werden handelsübliche oder aber für diesen Zweck speziell hergestellte flexible, permanent magnetisierbare und magnetisierte Kunststoff-Folien verwendet, die ausreichend flexibel sind. Im Gegensatz zu den Kunststoff-Folien, die für aufklebbare Magnetpflaster verwendet werden, ist es erfindungsgemäss nicht unbedingt erforderlich dass die Kunststoff-Folie als solche hautverträglich ist, insbesondere wenn sie noch mit einem weiteren Textilgewebe dauernd überzogen ist.

Da handelsübliche Magnetfolien im allgemeinen streifenförmig abwechselnd positiv und negativ magnetisiert sind und sich derartige Magnetisierungen als gut und wirksam erwiesen haben, können diese ohne weiteres eingesetzt werden. Prinzipiell ist es aber auch möglich, die positiven und negativen Magnetpole in anderer Weise abwechselnd anzuordnen und damit wirksame Magnetfelder zu erzeugen.

Ein wesentlicher Vorteil der erfindungsgemässen grossflächigen Magnetfolien ist darin zu sehen, dass sie ohne direkten Kontakt zur Haut auch stundenweise zur Anwendung kommen können und dadurch wesentlich leichter und bequemer handzuhaben sind als fest aufgeklebte Magnetpflaster. Bei Verwendung von ausreichend stark magnetisierten Folien und ausreichend dünnem Gewebe ist trotzdem gewährleistet, dass ausreichende Feldstärken auf den Körper und insbesondere die Haut einwirken können.

Eine weitere Ausführungsform der erfindungsgemässen Magnetfolien ist dadurch gekennzeichnet, dass eine Seite der Magnetfolien mit Textilgewebe beklebt ist, welches ganzflächig oder in Teilbereichen mit einem Klettverschluss versehen ist. Das Textilgewebe kann in üblicherweise mit Textilklebern auf die Magnetfolien aufgeklebt werden. Der Klettverschluss kann auf dieses Textilgewebe zuvor aufgenäht oder aufgeklebt sein. Vorzugsweise wird die Magnetfolie mit einem Textilgewebe beklebt, welches als selbstklebendes Gewebe mit Klettverschluss vorliegt. Ein derartiges Material lässt sich besonders leicht und einfach auf der Magnetfolie aufbringen und kann gewünschtenfalls sogar nach einiger Zeit erneuert werden, sofern die Magnetfolie noch ausreichend gut erhalten ist. Ein besonderer Vorteil der neuen Befestigung von Magnetfolien besteht darin, dass zunächst einmal keinerlei Klebstoff auf die Haut oder den Körper gebracht werden muss, so dass alle klebstoffbedingten Reizerscheinungen oder Allergien von vornherein ausgeschlossen werden können. Weiterhin ist es möglich, sowohl das Magnetpflaster als auch die elastischen Bänder mit Klettverschlüssen voneinander zu trennen und zu waschen oder zu reinigen. Dies macht die neue Befestigungsmethode besonders hygienisch und allergiefrei. Ein besonderer Vorteil der erfindungsgemässen Magnetfolie ist, dass sie durch das aufgeklebte Textilgewebe mechanisch stabilisiert werden, ohne ihre Elastizität zu verlieren. Die bisher beobachtete Brüchigkeit der Folie bei längerem Gebrauch wird erfindungsgemäss herabgesetzt oder ganz unterdrückt.

Von besonderer Bedeutung sind die neuen Magnetfolien und ihre Methode zur Befestigung in der Tiermedizin, wo insbesondere bei behaarten Tieren wie Pferden und Hunden selbstklebende Magnetfolien nicht zur Anwendung kommen können. Bei der Befestigung von Magnetfolien ohne Klebstoff mit Hilfe üblicher Bandagen, Bändern oder Klammern wurde bisher als sehr nachteilig empfunden, dass die Folien verrutschen oder sehr schnell brüchig werden und dadurch für eine Wiederverwendung kaum geeignet sind. Erfindungsgemässe Magnetfolien können daher mit besonderem Erfolg auch in der Tiermedizin eingesetzt werden. Darüberhinaus bietet sich ihre Anwendung an bei Sportverletzungen und der Behandlung bewegter und/oder behaarter Körperteile wie Gelenken, Armen, Beinen oder behaarten Kopfpartien.

Insbesondere die grossflächigen Magnetfolien, aber auch kleiner dimensionierte Magnetfolien mit einem Klettverschluss auf der Rückseite sind gut geeignet für die Therapie mit feuchter Wärme. Hierzu kann es nützlich sein, die der Haut zugewandte Seite des Textilgewebes, die vorzugsweise aus einem Baumwoll- oder Leinenflanell besteht, vor der Anwendung etwas anzufeuchten. Prinzipiell genügt aber auch die normale Luftfeuchtigkeit sowie die von der Haut abgesonderten Mengen an Feuchtigkeit, um unter der Folie feucht-warme Bedingungen zu schaffen, die lindernd und heilend wirken. Diese Ausführungsformen können erfindungsgemäss auch in einfacher Weise nur stundenweise zur Anwendung kommen. Man benötigt keine elektrischen Anschlüsse und vermeidet die Risiken elektrischer Geräte direkt am Körper. Während bei selbstklebenden Folien starke Feuchtigkeitsabsonderungen zusammen mit dem Klebstoff und den Reib- und Zerreffekten zu gelegentlichen Hautreizungen geführt hat, verstärkt die Anwesenheit der Feuchtigkeit im Textilgewebe die therapeutische Wirksamkeit der Magnetfolie.

Eine weitere Ausführungsform der erfindungsgemässen Magnetfolie, insbesondere für die Therapie mit feuchter Wärme besteht aus einer Magnetfolie, die in einen Flanellbezug eingesetzt ist, der wie ein Briefumschlag umklappbar bzw. einschlagbar ist. Eine derartige Magnetfolie mit Flanell kann mit einer elastischen Binde mit Klettverschluss am Körper befestigt werden. Diese Ausführungsform ist besonders geeignet, für behaarte und/oder stark bewegte Körperteile, die sich jedoch andererseits leicht mit einer elastischen Binde mit Klettverschluss umwickeln lassen.

Insbesondere bei Ausführungsformen, bei denen es auf eine Steigerung der Temperatur unterhalb der Folie ankommt, hat es sich bewährt, die Rückseite der magnetisierten Magnetfolie mit einer reflektierenden Metallfolie zu bekleben. Hierdurch wird die Wärmeabstrahlung verringert.

Eine weitere Ausführungsform der erfindungsgemässen Magnetfolie besteht aus einem Flanell-Lappen, auf denen die Magnetfolie aufgelegt wird und bei der diese beiden Teile zusammen mit einem üblichen Heftpflaster am Körper befestigt werden. Durch den Flanell-Lappen zwischen der Magnetfolie und der Haut wird der Effekt der feuchten Wärme verstärkt. Aus hygienischen Gründen sollte dieser Flanell-Lappen nach 12 bis 24 Stunden ausgewechselt werden. Prinzipiell ist es aber auch ohne weiteres möglich, den Flanellstoff und die Magnetfolie 48 bis 60 Stunden auf dem Körper zu belassen.

In den nachfolgenden Beispielen sind typische Ausführungsformen der erfindungsgemässen Magnetfolien näher erläutert:

Beispiel 1

Eine 1,5 mm starke flexible Magnetfolie, die streifenförmig abwechselnd magnetisiert ist, und zwar jeweils 4 mm magnetisiert, 1 mm unverändert und danach 4 mm in der Gegenrichtung magnetisiert (lieferbar beispielsweise von der Firma Rheinmagnet Horst Baermann GmbH, Neunkirchen) wird auf die Dimension 25×40 cm zugeschnitten. Dieses Material wird in ein dünnes Flanellgewebe eingenäht oder eingeklebt. Hierbei wird die magnetisierte Seite der Kunststoff-Folie durch eine farbige Bedruckung des Flanellgewebes auf der magnetisierten Seite gekennzeichnet. Das so erhaltene Produkt wird in eine darüber passende dünne Leinenhülle gesteckt, die mit einem Klettverschluss verschliessbar ist. Eine Seite dieses Bezuges ist ebenfalls farbig markiert. Beim Einlegen ist darauf zu achten, dass die farbigen Markierungen der Einlage und des Bezuges auf der selben Seite sind. Das so erhaltene Kissen wird mit der farbig markierten Seite im Bett unter den Rücken, das Kreuz oder den Hinterkopf gelegt, so dass die Magnetfelder in den Körper eindringen können. Das erfindungsgemässe Kissen kann auch beim Sitzen in einem Sessel oder einem Stuhl verwendet werden, um die untere Rückenpartie bei Kreuzschmerzen zu behandeln. Der abnehmbare Kissenbezug kann gewaschen und wiederverwendet werden.

Beispiel 2

Die gleiche Magnetfolie wie im Beispiel 1 wird auf der magnetisierten Seite mit einem weichen Flanellgewebe beklebt und in Grössen von 30×45 cm geschnitten. Die so erhaltenen Stücke werden in einen dazu passenden Leinenbezug mit Klettverschluss gepackt. Das Kissen wird mit der Seite, die durch das aufgeklebte Flanellgewebe sich wärmer anfühlt zum Körper hin verwendet. Dieses Kissen kann beispielsweise beim Autofahren zur Behandlung eines schmerzenden Kreuzes verwendet werden. Ein ähnliches Kissen kann auch im oberen Teil des Sitzes angebracht werden, um Schmerzen in der Nackengegend zu behandeln.

Beispiel 3

Eine 1,5 mm starke flexible Magnetfolie, die streifenförmig abwechselnd magnetisiert ist, und zwar jeweils 4 mm magnetisiert, 1 mm unverändert, danach 4 mm in der Gegenrichtung magnetisiert ist (lieferbar beispielsweise von der Firma Rheinmagnet Horst Baermann GmbH, Neunkirchen) wird auf die Dimension 5×8 cm zugeschnitten und auf der nichtmagnetisierten Seite mit einem selbstklebenden Textilband mit Klettverschluss beklebt. Die so erhaltene Magnetfolie kann in einfacher Weise mit elastischen Bändern mit Klettverschluss an den gewünschten Körperstellen befestigt werden, indem man sie mit der magnetisierten Seite zum Körper hin mit den Bändern festbindet. Die Magnetfolie rutscht nicht, liegt jedoch in der Weise am Körper an, dass sie ihre therapeutische Wirkung voll entfalten kann. Die Folie kann auf Wunsch jederzeit abgenommen und wieder aufgelegt werden. Sie kann leicht gereinigt werden. Die elastischen Bänder sind waschbar und können daher auch in sehr hygienischer Weise wieder verwendet werden. Selbstklebendes Textilband auf der Rückseite erhöht die Lebensdauer der Magnetfolie um ein Mehrfaches. Um einen direkten Kontakt der Magnetfolie mit der Haut zu vermeiden, wird vorzugsweise ein Flanell-Lappen mit den Abmessungen 5×8 cm oder etwas grösser zwischen die Magnetfolie und die Haut gelegt. Dieser Flanell-Lappen kann bei jeder Anwendung durch einen neuen ersetzt werden. Gewünschtenfalls werden diese Flanell-Lappen auch wieder gewaschen und wiederverwendet.

Beispiel 4

Eine 1,0 mm starke flexible Magnetfolie, die streifenförmig abwechselnd magnetisiert ist und zwar jeweils 4 mm magnetisiert, 1 mm unverändert, danach 4 mm in der Gegenrichtung magnetisiert ist (lieferbar beispielsweise von der Firma Rheinmagnet Horst Baermann GmbH, Neunkirchen) wird auf die Dimension 6×10 cm zugeschnitten. Auf der nichtmagnetisierten Seite ist die Magnetfolie mit einer reflektierenden Metallfolie überzogen. Diese Magnetfolie wird mit einem handelsüblichen Heftpflaster auf die Haut befestigt, jedoch wird um einen direkten Kontakt der Magnetfolie mit der Haut zu vermeiden, ein Flanell-Lappen mit den Abmessungen 6×10 cm oder etwas grösser zwischen die Magnetfolie und die Haut gelegt. Dieser Flanell-Lappen kann bei jeder Anwendung durch einen neuen ersetzt werden. Gewünschtenfalls werden diese Flanell-Lappen auch wieder gewaschen und wiederverwendet.

Beispiel 5

Die gleiche Magnetfolie wie im Beispiel 4 wird in ein Flanellsäckchen eingelegt, welches wie ein Briefumschlag umschlagbar und einlegbar ist. Diese Magnetfolie wird durch ein entsprechend grosses Stück Klettverschluss an einer elastischen Binde festgehalten und mit Hilfe der elastischen Binde mit Klettverschluss am Körper befestigt. Die Flanellsäckchen werden nach ca. 24 bis 48 Stunden gegen neue ausgetauscht. Gewünschtenfalls können die Flanellsäckchen gewaschen und wiederverwendet werden.

**Patentansprüche**

1. Magnetfolien mit streifenförmiger abwechselnder positiver und negativer Magnetisierung in Abständen von 1 bis 12 mm, vorzugsweise 3 bis 6 mm mit Feldstärken von 300 bis 1000 Gauss, dadurch gekennzeichnet, dass sie mindestens auf einer Seite ein Gewebe aus saugfähigem, hautverträglichem Textilgewebe aus Wolle, Baumwolle oder Leinen aufweisen.

2. Magnetfolien gemäss Anspruch 1, dadurch gekennzeichnet, dass das Textilgewebe Flanell ist.

3. Magnetfolien gemäss Anspruch 1 oder 2, dadurch gekennzeichnet, dass sie eine Fläche von 400 bis 1500 cm² aufweisen und allseitig mit einem oder mehreren Textilgeweben überzogen sind, von denen mindestens eines abnehmbar und waschbar ist.

4. Magnetfolien gemäss einem der Ansprüche 1 bis 3, dadurch gekennzeichnet, dass eine Seite der Magnetfolie mit Textilgewebe beklebt ist, welches ganzflächig oder in Teilbereichen mit Klettverschluss versehen ist.

5. Magnetfolie gemäss Anspruch 4, dadurch gekennzeichnet, dass das Textilgewebe als selbstklebendes Gewebe mit Klettverschluss vorliegt.

6. Magnetfolien gemäss einem der Ansprüche 1 bis 5, dadurch gekennzeichnet, dass die Rückseite mit einer reflektierenden Metallfolie versehen ist.

7. Verfahren zur Befestigung von Magnetfolien gemäss Anspruch 1 oder 2 an bewegten und/oder behaarten Körperteilen, dadurch gekennzeichnet, dass man die Magnetfolien auf einer Seite mit Textilgewebe beklebt, welches ganzflächig oder in Teilbereichen mit einem Klettverschluss versehen ist und die so erhaltenen Magnetfolien mit elastischen Bändern mit Klettverschlüssen auf die Körperteile bindet.

**Claims**

1. A magnetic sheet having an alternating stripe-shaped positive and negative magnetization at distances of from 1 to 12 mm, more preferably from 3 to 6 mm, with field strengths of from 300 to 1000 Gauss, characterized in that it has a fabric having an absorbing capacity and being compatible with the skin made of wool, cotton, or linen on at least one surface thereof.

2. The magnetic sheet according to claim 1, characterized in that the textile fabric is flannel.

3. The magnetic sheet according to claims 1 or 2, characterized in that it has a surface of from 400 to 1500 cm² and that it is covered on all sides thereof with one or several textile fabric(s) at least one of which is removable and launderable.

4. The magnetic sheet according to any one of claims 1 to 3, characterized in that one side of the magnetic sheet is laminated with a textile fabric which is provided with a hook and loop-type (Velcro®) strip fastener on the entirety or only part(s) of its surface.

5. The magnetic sheet according to claim 4, characterized in that the textile fabric is in the form of a self-adhesive fabric with Velcro strip fastener.

6. The magnetic sheet according to any one of claims 1 to 5, characterized in that the reverse side thereof is provided with a reflecting metal film.

7. A process for fixing a magnetic sheet according to claims 1 or 2 to parts in motion and/or hairy parts of the body, characterized in that the magnetic sheet is laminated on one surface thereof with a textile fabric which is provided with a hook and loop-type (Velcro®) strip fastener on the entirety or only part(s) of its surface and the magnetic sheet having been thus obtained is bound to the part of the body by means of elastic tapes having Velcro strip fasteners.

**Revendications**

1. Feuilles magnétiques à aimantation alternée positive et négative par bandes situées à intervalles de 1 à 12 mm, de préférence de 3 à 6 mm, avec des champs de 300 à 1000 gauss, caractérisées en ce qu'elles présentent au moins sur une face un tissu de laine, de coton ou de lin absorbant et compatible avec la peau.

2. Feuilles magnétiques selon la revendication 1, caractérisées en ce que le tissue est la flanelle.

3. Feuilles magnétiques selon l'une des revendications 1 et 2, caractérisées en ce qu'elles ont une surface de 400 à 1500 cm² et sont revêtues partout d'un ou de plusieurs tissus dont au moins un est amovible et lavable.

4. Feuilles magnétiques selon l'une des revendications 1 à 3, caractérisées en ce que sur une face de la feuille magnétique est collé un tissu qui est pourvu d'un ruban de fixation à auto-accrochage, à boucles et crochets adhérant par pression sur toute sa surface ou dans des parties.

5. Feuilles magnétiques selon la revendication 4, caractérisées en ce que le tissu est un tissu auto-adhésif avec ledit ruban à auto-accrochage.

6. Feuilles magnétiques selon l'une des revendications 1 à 5, caractérisées en ce que leur dos est pourvu d'une feuille métallique réfléchissante.

7. Procédé de fixation de feuilles magnétiques selon l'une des revendications 1 ou 2 à des parties du corps mobile et/ou velues, caractérisé en ce qu'on colle sur une face des feuilles magnétiques un tissu pourvu d'un ruban de fixation à auto-accrochage, à boucles et crochets adhérant par pression sur toute sa surface ou dans des parties de celle-ci, et on fixe les feuilles magnétiques ainsi obtenues sur les parties du corps avec des bandes élastiques à ruban de fixation à auto-accrochage, à boucles et crochets adhérant par pression.